Europäisches Patentamt

European Patent Office

Office européen des brevets

(19)

(11) Veröffentlichungsnummer: **0 243 854**
**B1**

(12) **EUROPÄISCHE PATENTSCHRIFT**

(45) Veröffentlichungstag der Patentschrift:
18.07.90

(51) Int. Cl.⁵: **A61M 5/14**, G01N 27/416

(21) Anmeldenummer: 87105853.3

(22) Anmeldetag: 21.04.87

(54) Implantierbare, abgleichbare Messvorrichtung für eine Körpersubstanz.

(30) Priorität: 02.05.86 DE 3614821

(43) Veröffentlichungstag der Anmeldung:
04.11.87 Patentblatt 87/45

(45) Bekanntmachung des Hinweises auf die Patenterteilung:
18.07.90 Patentblatt 90/29

(84) Benannte Vertragsstaaten:
DE FR GB IT

(56) Entgegenhaltungen:
EP-A- 0 036 171
EP-A- 0 102 548
WO-A-81/01794
US-A- 4 360 019
US-A- 4 557 722

(73) Patentinhaber: Siemens Aktiengesellschaft,
Wittelsbacherplatz 2, D-8000 München 2(DE)

(72) Erfinder: Franetzki, Manfred, Dr, Schleifweg 7b,
D-8525 Uttenreuth(DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine in einen Körper implantierbare Meßvorrichtung für eine Körpersubstanz mit einem in einem Gehäuse befindlichen Meßsensor, dem eine zu messende, über eine erste Membran als Filtrat aus dem Körper extrahierbare Körpersubstanz und außerdem eine über ein Durchstichseptum und eine Zuleitvorrichtung von außen injizierbare körperfremde Substanz, die eine Eich-, Spül- oder Wirksubstanz zuführbar sind.

Die Steuerung eines implantierten Medikamentendosiergerätes erfolgt im Idealfall über eine ebenfalls implantierte Meßvorrichtung, die den zu regelnden Körperparameter über einen Meßsensor erfaßt. Aus dem elektronisch verarbeiteten Meßsignal wird ein Steuersignal für eine Dosierpumpe abgeleitet. Ein solches Gerät ist beispielsweise aus der WO 81/01794 bekannt. Dort dient die Glucosekonzentration im Körper, zum Beispiel im Blut, im Bindegewebe oder im Peritoneum des Patienten, als Meß- bzw. Regelgröße. Steuergröße ist die Insulinförderrate. Bei einem solchen sogenannten Closed-Loop-Gerät ist der Meßsensor in der Regel einer Drift unterworfen, die sowohl den Nullpunkt, die Steigung als auch eventuell die Form der Meßcharakteristik betreffen kann.

Eine implantierbare Meßvorrichtung der vorgenannten Art ist aus der EP-A 0 102 548 bekannt. Bei dieser Meßvorrichtung steht der Meßsensor direkt oder über eine dazwischenliegende, für bestimmte Körpersubstanzen durchlässige Membran in Verbindung mit einer Körperflüssigkeit. Um den Sensor in einem wirksamen Betriebszustand zu halten, kann dieser in ein Wartungsgehäuse zurückgezogen und dort gereinigt oder regeneriert werden.

Darüber hinaus ist aus der EP-A 0 036 171 eine Meßvorrichtung der eingangs beschriebenen Art bekannt, deren Meßsensor fest in einem katheterähnlichen Rohr befestigt ist. Über einen Kanal innerhalb dieses Rohres kann der Sensor mit einer Flüssigkeit bekannter chemischer Eigenschaften beaufschlagt werden, um das Sensorausgangssignal zu kalibrieren. Dabei können nur solche Flüssigkeiten verwendet werden, die körperverträglich sind.

Der Erfindung liegt die Aufgabe zugrunde, eine Meßvorrichtung der eingangs genannten Art so auszubilden, daß deren Meßsensor über längere Zeit an der gleichen Stelle im Körper eines Patienten implantiert bleiben kann, ohne daß dieser verfälschte Meßsignale liefert. Dazu soll eine Drift im Meßsignal des Meßsensors mit Hilfe einer Eichflüssigkeit erfaßt und ausgeglichen werden können. Außerdem soll es möglich sein, mit Hilfe von Spülvorgängen Ablagerungen im Meßkreislauf ohne Ortsveränderung der zu reinigenden Elemente zu verhindern oder zu beseitigen.

Diese Aufgabe wird durch die im Patentanspruch 1 angegebene Erfindung gelöst. Dadurch ist erreicht, daß der Meßsensor der implantierten Meßvorrichtung ohne Eingriff in den Körper von außen und ohne den Meßsensor selbst aus seiner Lage im Körper entfernen zu müssen, zu den erforderlichen Zeitpunkten abgeglichen werden kann. Dazu wird in einem ersten Schritt die am Meßsensor befindliche Substanz abgesaugt, in einem zweiten Schritt Eichsubstanz an den Meßsensor gebracht und in einem dritten Schritt diese Eichsubstanz vom Meßsensor gemessen. Dabei ist es besonders vorteilhaft, daß das Durchstichseptum und der Pufferspeicher über Flußpfade mit dem Meßsensor verbunden sind. Durch die Anordnung des Pufferspeichers vom Durchstichseptum her betrachtet hinter dem Meßsensor ist darüber hinaus die Möglichkeit geschaffen, eine Eichsubstanz an den Meßsensor heran und durch diesen hindurchzuführen. Dies bietet die Möglichkeit, das für die Eichsubstanz zu erwartende Ergebnis mit dem tatsächlichen Meßwert des Meßsensors zu vergleichen. Im Falle einer unzulässigen Drift kann diese von einem äußeren Gerät mit nicht dargestellten Mitteln abgeglichen werden. Der dazu notwendige Austausch an Informationen zwischen der Meßvorrichtung und dem äußeren Gerät geschieht mit Hilfe von auch bei implantierten Dosiergeräten für die medizinische Anwendung bekannten drahtlosen Übertragungsmitteln. Anstelle der Eichsubstanz kann auch eine Spülflüssigkeit injiziert werden, die in der Lage ist, Ablagerungen auf dem Meßsensor, im Flußpfad oder im Pufferspeicher abzulösen, so daß diese durch Absaugen der Spülflüssigkeit entfernt werden können.

In einer vorteilhaften Ausgestaltung der Erfindung nach Patentanspruch 2 kann darauf verzichtet werden, zum Abgleich des Meßsensors das gesamte im Pumpenkreislauf befindliche Ultrafiltrat durch die von außen zugeführte Substanz auszuspülen. Vielmehr ist der Meßsensor Bestandteil eines eigenen, durch eine semipermeable Membran vom Kreislauf des Ultrafiltrats getrennten Flüssigkeitskreislaufs, in dem Eich- oder Spülvorgänge unabhängig vom Kreislauf des Ultrafiltrats durchgeführt werden können.

Falls gemäß Patentanspruch 3 als körperfremde Substanz ein flüssiges Enzym benutzt werden soll, bedeutet dies, daß diese Substanz in erheblich geringerer Menge zugeführt werden kann, als dies bei einer unmittelbaren Zuführung in den Trakt des Ultrafiltrats notwendig wäre. Dabei verhindert die doppelte Ultrafilterung ein Austreten des im Körper toxisch wirkenden Enzyms in den Körperkreislauf.

Weitere Vorteile und Ausgestaltungen der Erfindung ergeben sich aus der Beschreibung von Ausführungsbeispielen anhand der Figuren. Es zeigen:

Fig. 1 eine Meßvorrichtung für ein Ultrafiltrat mit integrierter Abgleichvorrichtung;

Fig. 2 eine Meßvorrichtung für ein Dialysat mit integrierter Abgleichvorrichtung;

Fig. 3 eine Meßvorrichtung für ein Ultrafiltrat mit einem Enzymsensor mit integrierter Abgleichvorrichtung; und

Fig. 4 eine Meßvorrichtung für ein Dialysat mit Enzymsensor mit integrierter Abgleichvorrichtung.

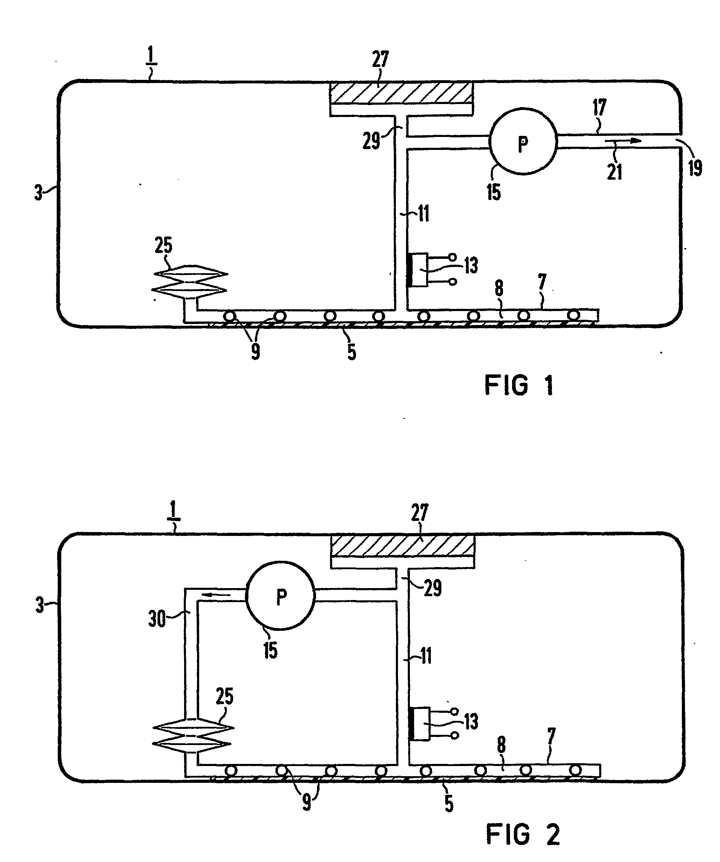

In Fig. 1 ist eine in den Körper eines Patienten implantierbare Meßvorrichtung 1 dargestellt, die zur Messung oder Analyse einer Körperflüssigkeit, insbesondere von Glucose in der Körperflüssig-

keit, vorgesehen ist. Die Meßvorrichtung 1 umfaßt ein Gehäuse 3, welches relativ flach ausgebildet ist. Eine Gehäusefläche, z.B. eine Stirnfläche des Gehäuses 3, ist mit einer großflächigen semipermeablen Membran 5 versehen. Die Membran 5 hat eine Fläche von z.B. 20 cm². Die Membran 5 ist von einer flachen, topfförmigen Kapsel 7 eingefaßt. Im Zwischenraum 8 zwischen der Kapsel 7 und der Membran 5 ist ein Stützgitter 9 angeordnet. Eine Verbindung oder Zuleitung 11 führt von dem Zwischenraum 8, der von der Membran 5 und der Kapsel 7 gebildet wird, über einen Meßsensor 13 zu einer Fördereinrichtung 15, z. B. einer elektrischen Pumpe P. Von der Fördereinrichtung 15 führt eine weitere Leitung 17 zu einer Auslaßöffnung 19 im Gehäuse 3. Dem Meßsensor 13 ist eine (nicht gezeigte) Elektronik zur Meßwertaufbereitung und/oder -verarbeitung nachgeschaltet.

An den Zwischenraum 8 zwischen der Membran 5 und der Kapsel 7 ist bevorzugt ein Pufferspeicher 25 angeschlossen. Der Pufferspeicher 25 kann beispielsweise ein Balg mit elastischen Wänden sein, der in sich zusammenfallen kann. Es ist auch möglich, den Pufferspeicher durch eine sonstige Elastizität im Fluidtrakt zu bilden, etwa durch die Eigenelastizität der Membran 5 oder der Kapsel 7.

Ein Durchstichseptum 27 ist über einen Verbindungssteg 29 mit der Zuleitung 11 verbunden. Es entsteht so ein Flußpfad, bestehend aus Zuleitung 11, Zwischenraum 8 und Verbindungssteg 29, welcher den Pufferspeicher 25, den Meßsensor 13 und das Durchstichseptum 27 leitungsmäßig miteinander verbindet.

Mit Hilfe der Fördereinrichtung 15 wird von der Rückseite der Membran 5 ein dort entstandenes Ultrafiltrat über den Meßsensor 13 transportiert und durch die Auslaßöffnung 19 wieder in den Körper des Patienten zurückgeführt.

Die semipermeable Membran 5 ist so ausgewählt, daß nur Substanzen mit einem Molekulargewicht unterhalb eines vorgegebenen Grenzwertes als Ultrafiltrat passieren können. Der vorgegebene Grenzwert richtet sich dabei nach der zu messenden oder zu analysierenden Substanz. Insbesondere ist die Membran 5 so bemessen, daß sie im wesentlichen für Moleküle bis zur Größe von Glukosemolekülen durchlässig ist. Die Fläche der Membran 5 ist so groß gewählt, daß selbst dann, wenn die Membran 5 mit Bindegewebe vollständig beschichtet sein sollte, ein durch die Fördereinrichtung 15 eingeprägter mittlerer Förderfluß des Filtrats aufrechterhalten werden kann. Dieses ist möglich, da das Bindegewebe stets vom Körper versorgt wird und so eine endliche Durchlässigkeit beibehält. Die abgepumpte Filtratmenge muß dabei so gering sein, daß die Gewebediffusionskapazität den Zufluß nicht behindert. Der Einfluß der Permeabilität der Membran 5 auf das vom Meßsensor 13 ermittelte Meßsignal ist damit ausgeschaltet.

Zum Einleiten des Abgleichens des Meßsensors 13 wird die Nadel oder Kanüle einer Spritze durch das Durchstichseptum 27 von außen her durchgestochen. Die in dem Flußpfad enthaltene Flüssigkeit wird durch die Kanüle so lange abgesaugt, bis der Pufferspeicher 25 zusammengefallen ist. Anschließend wird die Spritze außerhalb des Patienten entleert und mit einer Eichsubstanz gefüllt. Die Spritze mit der Eichsubstanz wird durch das Durchstichseptum 27 hindurchgestochen und die Eichsubstanz in den Flußpfad entleert. Es wird so lange Eichsubstanz eingeführt, bis der Raum bis zum Meßsensor 13 vollständig gefüllt ist. Dadurch ist sichergestellt, daß am Ort des Meßsensors 13 Eichsubstanz vorliegt. Entsprechend dem vom Meßsensor 13 gelieferten Meßwert wird der Meßsensor 13 samt nachfolgender Elektronik sodann abgeglichen. Der Abgleich wird dabei von außen über an sich bei implantierbaren Geräten bekannte drahtlose Kommunikationsmöglichkeiten, wie z.B. eine elektromagnetische Signalübertragung mit Hilfe von Antennen (wie Spulen), vorgenommen. Auf diese Weise ist ein Abgleich des Meßsensors 13 im implantierten Zustand ohne direkten Eingriff von außen möglich. Dabei wird beispielsweise die Verstärkung (Empfindlichkeit) oder aber der Nullpunkt der nachgeschalteten Elektronik so nachgestellt, daß einer bestimmten (bekannten) Konzentration der Eichsubstanz wieder ein vorgegebenes Ausgangssignal entspricht. Das Abgleichen kann - abweichend hiervon - auch darin bestehen, daß eine veränderte tabellarische Zuordnung von Konzentration und Ausgangssignal ermittelt, festgehalten, aufgezeichnet und/oder dargestellt wird, und zwar außerhalb des Patienten.

In Fig. 2 ist ein Ausführungsbeispiel gezeigt, welches statt eines Ultrafiltrats dem Meßsensor 13 ein Dialysat zugeführt wird. Gleiche Teile sind mit gleichen Bezugszeichen versehen. Der Aufbau ist mit dem gemäß Fig. 1 identisch bis auf die Ausnahme, daß keine weitere Leitung 17 und keine Auslaßöffnung 19 an die Fördereinrichtung 15 anschließen. Stattdessen ist die Fördereinrichtung 15 über eine Leitung 30 mit dem Pufferspeicher 25 verbunden. Es entsteht so ein geschlossener Kreislauf, welcher über die Leitung 30, den Pufferspeicher 25, den Zwischenraum 8 zwischen Membran 5 und Kapsel 7 und die Zuleitung 11 zurück zur Fördereinrichtung 15 führt. An der Zuleitung 11 ist der Meßsensor 13 angeschlossen. Ein Ansaugen der Körperflüssigkeit in Richtung auf die Membran 5 durch die Fördereinrichtung 15 findet hier - im Gegensatz zur Vorrichtung nach Fig. 1 - nicht statt. In diesem geschlossenen Kreislauf kann außer dem Dialysat auch ein anfangs eingebrachtes Enzym (dieses ist also dann nicht am Meßsensor 13 gebunden) herumgepumpt werden.

Die Membran 5 ist hier so ausgelegt, daß ein Konzentrationsausgleich zwischen der Flüssigkeit im Körpergewebe auf der Vorderseite der Membran 5 einerseits und der Substanz im Zwischenraum 8 zwischen Rückseite der Membran 5 und Kapsel 7 andererseits stattfindet. Eine solche Vorgehensweise ist an sich aus der Dialysetechnik bekannt. Die Konzentrationen der für die Membran 5 durchlässigen Substanzen im Flußpfad 11 der Meßeinrichtung 1 gleichen sich mit denen im Körpergewebe auf der Vorderseite der Membran 5 aus. Das Signal des Meßsensors 13 entspricht damit der aktuellen Konzentration der Meßsubstanz im Körper; es tritt nur zeitlich verzögert auf lediglich um die Transportzeit, die zum Transport von der Membran 5 bis zum Meßsensor 13 benötigt wird.

Auch in dieser Ausführungsform geschieht das Abgleichen entsprechend dem in Fig. 1 geschilderten Vorgang. Mit Hilfe der Kanüle einer Spritze wird zuerst die Substanz aus dem Flußpfad herausgesaugt, bis der Pufferspeicher 25 vollständig entleert und in sich zusammengefallen ist. Anschließend wird über dieselbe, im Patienten belassene Kanüle eine Eichsubstanz in den Flußpfad eingebracht. Die Anordnung des Pufferspeichers 25 hinter dem Sensor 13 stellt dabei sicher, daß am Ort des Meßsensors 13 Eichsubstanz vorhanden ist. Bei Feststellung einer unzulässigen Drift des Meßwerts des Meßsensors 13 wird dieser auf an sich bekannte Weise neu abgeglichen, z.B. drahtlos neu eingestellt oder aber mit einer neuen tabellarischen Zuordnung (Signal/Konzentration) versehen.

Für den Fall, daß der Meßsensor 13 zur Analyse eine Wirksubstanz benötigt, die verbraucht wird oder ihre Aktivität verliert, kann die verbrauchte oder abgenutzte Wirksubstanz über das Durchstichseptum 27 aus dem Flußpfad 29, 11 und dem Pufferspeicher 25 entfernt und durch eine neue, d.h. frische gleichartige Wirksubstanz ersetzt werden. Bei dieser Wirksubstanz kann es sich zum Beispiel wieder um ein Enzym handeln, das der Meßsensor 13 für die Aufrechterhaltung seiner Funktion benötigt, das am Meßsensor 13 aber nicht fest gebunden ist und im Trakt oder Flußpfad 11, 29, 30 mit dem Dialysat umgepumpt wird. Ebenfalls können auf diese Weise Spülvorgänge vorgenommen werden, die Ablagerungen auf dem Meßsensor 13, im Flußpfad 29, 11 oder im Pufferspeicher 25 entfernen. Ist der Meßsensor 13 beispielsweise durch eine bei der Messung oder Analyse entstandene Substanz "vergiftet", d.h. unbrauchbar geworden, so kann durch eine geeignete Spülsubstanz, die beim Spülvorgang verwendet wird, eine Regenerierung des Meßsensors 13 erfolgen. Dazu wird zuerst die dem Meßsensor 13 befindliche Substanz mittels einer Spritze oder Kanüle abgesaugt. Dann wird über die Spritze eine Spülsubstanz eingebracht. Die Wirksubstanz und/oder Spülsubstanz kann anschließend wieder durch Absaugen entfernt werden.

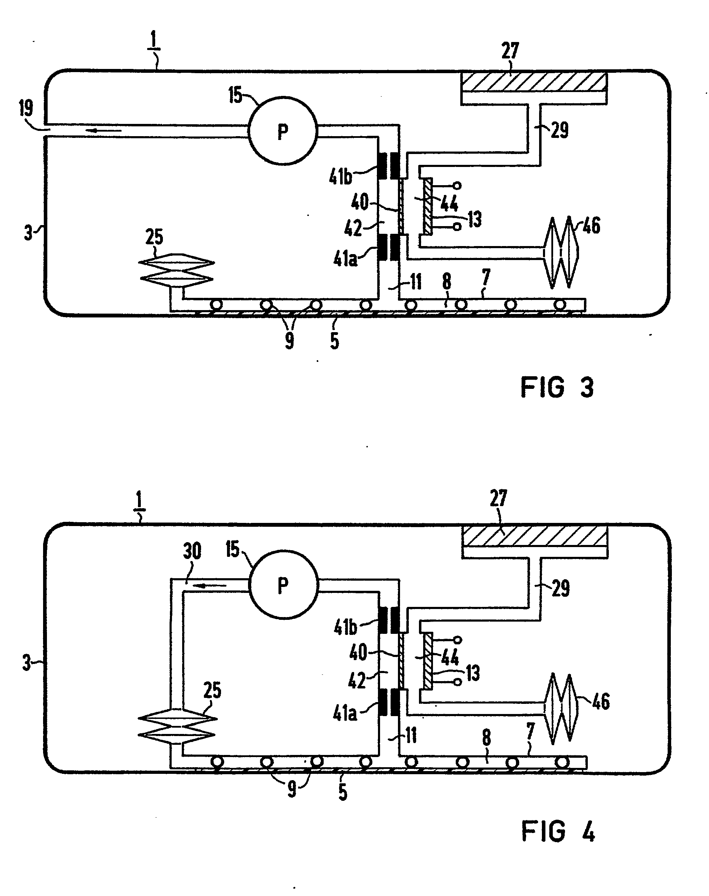

In Fig. 3 und 4 sind zwei weitere Ausführungsbeispiele einer implantierbaren Meßvorrichtung gezeigt, bei denen der Meßsensor insbesondere ein Enzymsensor ist. Fig. 3 unterscheidet sich von Fig. 1 dadurch, daß im Verlauf der Zuleitung 11 statt des Meßsensors 13 eine weitere semipermeable Membran 40 angeordnet ist. Zusätzlich befinden sich in der Zuleitung 11 vor und hinter der Membran 40 je eine Drosselungs- oder Strömungsengstelle 41a bzw. 41b. Dadurch wird an der Membran 40 eine Kammer 42 gebildet. Gegenüber dieser Kammer 42 befindet sich eine weitere Kammer 44, die von der Kammer 42 durch die Membran 40 getrennt ist. An der der Membran 40 gegenüberliegenden Seite dieser Kammer 44 befindet sich der Meßsensor 13. Hier ist die Kammer 44 über den Flußpfad 29 mit dem Durchstichseptum 27 und mit einem Pufferspeicher 46 verbunden. Bei dem Meßsensor 13 handelt es sich um einen Enzymsensor mit einem fixierten Enzym. Es kann sich auch um einen Enzymsensor handeln, bei dem der gesamte Trakt 29, 44 und 46 mit einem flüssigen Enzym gefüllt ist.

Das über die Membran 5 gewonnene Ultrafiltrat mit der zu messenden Körpersubstanz wird mit der Fördereinrichtung 15 zur Kammer 42 transportiert. Durch Konzentrationsausgleich geht die zu messende Körpersubstanz von der Kammer 42 über die semipermeable Membran 40 in die Kammer 44 über. Der Meßsensor 13 liefert ein Signal, das der Konzentration der zu messenden Körpersubstanz in der Kammer 44 entspricht.

Die Variante nach Fig. 3 zeichnet sich dadurch aus, daß nicht der gesamte Trakt des Ultrafiltrats durch eine von außen zugeführte Substanz gespült und der Meßsensor 13 damit abgeglichen wird, sondern daß nur die Kammer 44 unmittelbar vor dem Meßsensor 13 über das Septum 27 zugänglich ist und - wie geschildert - gespült wird. Im Falle der Verwendung eines Enzymsensors mit flüssigem Enzym wird bei diesem Vorgang insbesondere das Enzym ersetzt oder ein fixiertes Enzym regeneriert.

Des weiteren ergibt sich bei allen die Meßsubstanz verbrauchenden Systemen, insbesondere bei einem Enzymsensor oder elektrokatalytischen Sensor, noch eine weitere Möglichkeit des Nullpunktsabgleichs. Bei diesen Sensoren wird Glukose z.B. in Gluconsäure verbrannt.

Zum Nullpunktsabgleich des Meßsensors 13 wird die Fördereinrichtung 15 so lange ausgeschaltet, bis die Meßsubstanz vollständig verbraucht ist. Das Signal, das nun vom Meßsensor 13 abgegeben wird, entspricht dem Nullpunktssignal und kann zum Nullpunktsabgleich des Systems verwendet werden. Die Strömungsengstellen 41a, 41b sorgen dafür, daß die Diffusion in die Kammer 42 ausreichend gering wird.

Ein Vorteil dieses Ausführungsbeispiels ist die Verringerung des Verbrauchs an Enzym, da die Reaktion, d.h. der Verbrauch an Enzym, nur unmittelbar nach Füllen der Kammer 42 erfolgt und zwischen den Messungen das System ruht.

Der Vorgang des Abgleichens des Meßsensors 13 entspricht dem in Fig. 1 beschriebenen.

In Fig. 4 ist ein Ausführungsbeispiel gezeigt, das statt eines Ultrafiltrats über die Membran 5 ein Dialysat gewinnt. Der Kreislauf des Dialysats entspricht dem in Fig. 2 beschriebenen, die Anordnung des Meßsensors 13 und der Membran 40 entspricht dem in Fig. 3 beschriebenen Ausführungsbeispiel. Bei dieser Ausführungsform geschieht das Abgleichen entsprechend dem in Fig. 1 geschilderten Vorgang. Ebenso kann ein Nullpunktsabgleich des Meßsensors 13 durchgeführt werden, wie in Fig. 3 beschrieben.

Bei den in Fig. 3 und 4 beschriebenen Ausführungsbeispielen können Enzymsensoren eingesetzt werden, bei denen sich das Enzym in flüssiger Form am Meßsensor 13 befindet. Die Membran 40 in Fig. 3 bzw. die Membranen 40 und 5 in Fig. 4 verhindern ein Austreten des Enzyms aus der Meßvorrichtung in den Körper des Patienten, wo es toxisch wirken kann. Bei der Ausführungsform nach Fig. 4 ist durch die zwei Membranen 5 und 40 sogar ein doppelter Schutz vorhanden.

## Patentansprüche

1. In einen Körper implantierbare Meßvorrichtungen (1) für eine Körpersubstanz mit einem in einem Gehäuse (3) befindlichen Meßsensor (13), dem die zu messende, über eine erste Membran (5) als Filtrat aus dem Körper extrahierbare flüssige Körpersubstanz und außerdem eine über ein Durchstichseptum (27) und eine Zuleitvorrichtung (29, 11) von außen injizierbare körperfremde flüssige Substanz, die eine Eich-, Spül- oder Wirksubstanz ist, zuführbar sind, dadurch gekennzeichnet, daß die erste Membran (5) von einer topfförmigen Kapsel (7) eingefaßt ist und der von dieser gebildete Raum (8) hinter der ersten Membran (5), der Meßsensor (13), das Durchstichseptum (27) und ein erster eigenelastischer Pufferspeicher (25) flüssigkeitsleitend miteinander verbunden ist, daß eine Fördereinrichtung (15) vorhanden ist, die aus dem Körper extrahierte flüssige Körpersubstanz durch den Meßsensor (13) fördert, und die Fördereinrichtung (15) zwischen dem Raum (8) und einer Austrittsöffnung (19) im Gehäuse (3) oder in einem geschlossenen, aus dem Raum (8) hinter der ersten Membran (5), der Fördereinrichtung (15) selbst und dem ersten eigenelastischen Pufferspeicher (25) gebildeten Flüssigkeitskreislauf angeordnet ist, und daß das Durchstichseptum (27) über einen Verbindungssteg (29) in eine Zuleitung (11) zwischen dem Raum (8) und der Fördereinrichtung (15) einmündet, wobei die Kapsel (7), der Meßsensor (13), die Fördereinrichtung (15), das Durchstichseptum (27) und der erste Pufferspeicher (25) in dem gemeinsamen implantierbaren Gehäuse (3) untergebracht sind.

2. Meßvorrichtung nach Anspruch 1, dadurch gekennzeichnet, daß in der Zuleitung (11) zwischen dem Raum (8) hinter der ersten Membran (5) anstelle des Meßsensors (13) eine zwischen zwei Drosselungs- oder Strömungsengstellen (41a; 41b) angeordnete erste Kammer (42) mit einer zweiten Membran (40) angeordnet ist, deren Außenseite von einer zweiten, den Meßsensor (13) enthaltenden Kammer (44) flüssigkeitsdicht umschlossen ist, die sowohl mit dem Durchstichseptum (27) wie mit einem zweiten elastischen Pufferspeicher (46) flüssigkeitsleitend verbunden sind, wobei die erste Kammer (42), die zweite Kammer (44) und der zweite Pufferspeicher (46) ebenfalls in dem gemeinsamen implantierbaren Gehäuse (3) untergebracht sind.

3. Meßeinrichtung nach Anspruch 2, dadurch gekennzeichnet, daß als körperfremde flüssige Substanz ein flüssiges Enzym benutzt ist.

## Claims

1. Measuring devices (1) for a body substance, which can be implanted in a body, having a measuring sensor (13) located in a housing (3), to which measuring sensor can be supplied both the liquid body substance to be measured, which can be extracted as a filtrate from the body by way of a first membrane (5), and also a liquid substance foreign to the body which can be injected from outside by way of a pierceable septum (27) and a delivery device (29, 11), which liquid substance is a calibrating, rinsing or active substance, characterised in that the first membrane (5) is enclosed by a cup-shaped capsule (7), and the space (8) behind the first membrane (5) formed by this capsule, the measuring sensor (13), the pierceable septum (27) and a first inherently elastic buffer reservoir (25) are connected together in liquid-conducting manner, in that a conveying mechanism (15) is present which conveys through the measuring sensor (13) the liquid body substance extracted from the body, the conveying mechanism (15) being arranged between the space (8) and a discharge opening (19) in the housing (3) or in a closed liquid cycle formed from the space (8) behind the first membrane (5), the conveying mechanism (15) itself, and the first inherently elastic buffer reservoir (25), and in that the pierceable septum (27) opens by way of a connecting bridge (29) into a delivery line (11) between the space (8) and the conveying mechanism (15), with the capsule (7), the measuring sensor (13), the conveying mechanism (15), the pierceable septum (27) and the first buffer reservoir (25) being accommodated in the common, implantable housing (3).

2. Measuring device according to claim 1, characterised in that, in the delivery line (11), between the space (8) behind the first membrane (5), in place of the measuring sensor (13) is arranged a first chamber (42) with a second membrane (40), which first chamber (42) is arranged between two throttling or flow restriction points (41a; 41b), the outside of which second membrane (40) is surrounded in fluid-tight manner by a second chamber (44) containing the measuring sensor (13), which second chamber (44) is connected in liquid-conducting manner both to the pierceable septum (27) and to a second elastic buffer reservoir (46), the first chamber (42), the second chamber (44) and the second buffer reservoir (46) being likewise accommodated in the common, implantable housing (3).

3. Measuring device according to claim 2, characterised in that a liquid enzyme is used as the liquid substance foreign to the body.

## Revendications

1. Appareil de mesure (1), implantable dans l'organisme et destiné à mesurer une substance de l'organisme, comprenant un capteur de mesure (13) se trouvant dans un boîtier (3) et auquel peuvent être amenées la substance liquide de l'organisme à mesurer, qui peut être extraite de l'organisme à travers une première membrane (5) sous la forme d'un filtrat et, en outre, une substance liquide, étrangère à l'organisme, qui est une substance d'étalonnage, de rinçage ou active et qui peut être injectée de l'extérieur par un septum perforable (27) et par un dispositif d'amenée (29, 11), caractérisé en ce que la première membrane (5) est bordée par une capsule (7) en forme de godet et l'espace (8) formé par celle-ci derrière la première membrane (5), le capteur de mesure (13), le septum (27) perforable et un premier réservoir-tampon (25) ayant une élasticité qui lui est propre, communiquent entre eux pour le passage d'un liquide, en ce qu'il est prévu un dispositif de mise en circulation (15) qui envoie la substance liquide de l'organisme qui en est extraite dans le capteur de mesure (13) et le dispositif de mise en circulation (15) est disposé entre l'espace (8) et un orifice de sortie (19) ménagé dans le boîtier (3) ou dans un circuit fermé pour du liquide formé de l'espace (8) derrière la première membrane (5), du dispositif de mise en circulation (15) lui-même et du premier réservoir-tampon ayant une élasticité qui lui est propre, et en ce que le septum (27) perforable débouche, en passant par un ajutage (29), dans un circuit d'amenée (11) entre l'espace (8) et le dispositif de mise en circulation (15), la capsule (7), le capteur de mesure (13), le dispositif de mise en circulation (15), le septum (27) perforable et le premier réservoir-tampon (25) étant logés dans le boîtier (3) commun et implantable.

2. Appareil de mesure suivant la revendication 1, caractérisé en ce que, à la place du capteur de mesure (13), est montée, dans le conduit (11) et entre l'espace (8) derrière la première membrane (5), une première chambre (42) disposée entre deux endroits d'étranglement ou de resserrement de l'écoulement (41a, 41b) et ayant une seconde membrane (40) dont la face extérieure est entourée d'une manière étanche aux liquides d'une seconde chambre (44) contenant le capteur de mesure (13), et communiquant, pour le passage d'un liquide, à la fois avec le septum (27) perforable et avec un second réservoir-tampon (46) élastique, la première chambre (42), la seconde chambre (44) et le second réservoir-tampon (46) étant logés également dans le boîtier (3) commun et implantable.

3. Appareil de mesure suivant la revendication 2, caractérisé en ce qu'une enzyme liquide est utilisée comme substance liquide étrangère à l'organisme.

FIG 1

FIG 2

FIG 3

FIG 4